# EUROPÄISCHE PATENTANMELDUNG

(11) **EP 0 562 241 A1**
(43) Veröffentlichungstag der Anmeldung: **29.09.1993**
(21) Anmeldenummer: 93101497.1
(22) Anmeldetag: 01.02.1993
(51) Int. Cl.: A61F 2/66

(54) **Künstlicher Fuss**

(30) Priorität: 27.03.1992 DE 4209974
(71) Anmelder: Otto Bock Orthopädische Industrie Besitz- und Verwaltungs-Kommanditgesellschaft, D-37115 Duderstadt (DE)
(72) Erfinder: Hiemisch, Christian, W-3408 Duderstadt (DE)
(74) Vertreter: Gramm, Werner, Prof., Dipl.-Ing.

(57) **Zusammenfassung**

Die Erfindung betrifft einen künstlichen Fuß, der im wesentlichem aus einem inkompressiblen Kern (1), einem Fersenkeil (2), einem Innenfuß (3) und einem alles umschließenden Außenfuß (5) besteht.

Zur Erschaffung eines komfortbetonten Prothesenfußes für normales Gehen werden erfindungsgemäß zusätzlich folgende Merkmale vorgeschlagen:
a) ein allseitig bewegliches, tiefliegendes Gelenk (8,9,10,11);
b) das Gelenk weist einen angenähert in der Lotrechten stehenden Bolzen (8) auf, dessen unteres Ende als Gelenkkopf (9) ausgebildet ist, der auf seiner Oberseite sowie seitlich von einer Lagerschale (10) übergriffen wird;
c) der Bolzen (8) stützt sich mit seiner der Ferse zugewandten Rückseite an einem horizontal gerichteten Plantarpuffer (21) ab, der in eine zur Anschlußfläche (4) und nach vorne offene Ausnehmung (22) im Kern (1) eingelegt ist und den Bolzen (8) nach Art einer im wesentlichen horizontal wirkenden Druckfeder beaufschlagt;
d) das obere Ende des Bolzens (8) ist an einem Adapter (14) befestigt, der sich mit seiner Unterseite in dem vor dem Bolzen (8) liegenden vorderen Bereich direkt oder indirekt auf dem Kern (1) und mit dem hinter dem Bolzen (8) liegenden hinteren Bereich auf dem Plantarpuffer (21) abstützt.

## Beschreibung

Die Erfindung betrifft einen künstlichen Fuß mit
- einem praktisch inkompressiblen, im Knöchelbereich eine obere Anschlußfläche bildenden Kern, der sich mit seiner an die Anschlußfläche anschließenden Vorderseite über einen nach vorn in der Höhe abnehmenden Ansatz in den Spannbereich des Fußes erstreckt und insgesamt etwa halb so lang ist wie der gesamte Fuß;
- einem sich an die Unterseite des Kernes anschließenden Fersenkeil aus weichem Kunststoffschaum;
- einem sich an die Vorderseite des Kernes und an die Vorderseite des Fersenkeils anschließenden Innenfuß, der sich bis in den Zehenbereich erstreckt und aus einem Kunststoffschaum besteht, und mit
- einem den Innenfuß mit Ausnahme der oberen Anschlußfläche vollständig umgebenden Außenfuß, der aus einer weichen, leicht verformbaren, hautbildenden Kunststoffschaumschicht besteht, deren Rückstellkräfte klein sind gegenüber den Rückstellkräften des Innenfußes.

Eine entsprechende Ausführungsform ist in der DE-PS 36 44 613 beschrieben. Es handelt sich um einen gelenklosen Fuß, der mit einem sich auf der Oberseite des Kerns abstützenden Anschluß-Bauteil in üblicher Weise lösbar verbindbar ist mittels eines von der Fußunterseite in eine Bohrung des Fußkerns eingeführten und in ein Muttergewinde des Anschluß-Bauteils eingeschraubten Gewindebolzens. Die Vorderseite des Fersenkeils schließt etwa mit der Vorderseite des Kerns ab, wobei der Fersenkeil bis über die halbe Fußhöhe hinaus nach hinten ansteigt. Der Innenfuß liegt ausschließlich an der Vorderseite des Kerns und der Vorderseite des Fersenkeils an. Der Außenfuß umschließt den Innenfuß sowie die Unter- und Rückseite des Fersenkeils und bildet in der genannten Bohrung eine dünne Abdeckschicht. Bei diesem vorbekannten künstlichen Fuß werden die Laufeigenschaften in der zweiten Phase der Abrollbewegung (beim Anheben der Ferse) praktisch ausschließlich von den flexiblen Eigenschaften des Innenfußes bestimmt. Der Außenfuß hat nur noch die Aufgabe des Schutzes vor eindringendem Wasser und vor eindringenden aggressiven Agenzien sowie der kosmetischen Ausbildung der Fußoberfläche.

Der Erfindung liegt die Aufgabe zugrunde, einen komfortbetonten Prothesenfuß für normales Gehen zu schaffen.

Ausgehend von dem eingangs beschriebenen künstlichen Fuß wird diese Aufgabe gemäß der Erfindung durch folgende Merkmale gelöst:
a) ein allseitig bewegliches, tiefliegendes Gelenk;
b) das Gelenk weist einen angenähert in der Lotrechten stehenden Bolzen auf, dessen unteres Ende als Gelenkkopf ausgebildet ist, der auf seiner Oberseite sowie seitlich von einer Lagerschale übergriffen wird;
c) der Bolzen stützt sich mit seiner der Ferse zugewandten Rückseite an einem horizontal gerichteten Plantarpuffer ab, der in eine zur Anschlußfläche und nach vorne offene Ausnehmung im Kern eingelegt ist und den Bolzen nach Art einer im wesentlichen horizontal wirkenden Druckfeder beaufschlagt;
d) das obere Ende des Bolzens ist an einem Adapter befestigt, der sich mit seiner Unterseite in dem vor dem Bolzen liegenden vorderen Bereich direkt oder indirekt auf dem Kern und mit dem hinter dem Bolzen liegenden hinteren Bereich auf dem Plantarpuffer abstützt.

Ein im Aufbau besonders einfaches Gelenk wird dann erreicht, wenn die Lagerschale in eine entsprechende, nach unten hin offene Ausnehmung im Kern eingesetzt ist und mit der Unterseite des Kerns angenähert bündig abschließt, und ferner dadurch, daß der Gelenkkopf durch eine halbkreisförmige Scheibe gebildet ist, die in der Längsmittelebene des Fußes liegt und mit ihrer Flachseite auf einem ebenen Abschnitt des Innenfußes aufliegt.

Zur Begrenzung der Schwenkbewegung des Bolzens ist es vorteilhaft, wenn der Bolzen von unten durch eine Durchtrittsöffnung in der Lagerschale und durch eine sich hieran anschließende Durchtrittsöffnung im Kern gesteckt ist, wobei die beiden miteinander fluchtenden Durchtrittsöffnungen sich nach oben konisch erweitern derart, daß bei lotrecht ausgerichtetem Bolzen zwischen dessen vorderen Umfangsbereich und den vorderen Begrenzungen der Durchtrittsöffnungen ein schmaler lichter Keilabstand, zwischen dem hinteren Bolzen-Umfangsbereich und den hinteren Begrenzungen der Durchtrittsöffnungen hingegen ein breiterer lichter Keilabstand verbleibt.

Bei dem erfindungsgemäßen Fuß ist der konstruktiv vorgesehene dorsale Schwenkwinkel (das ist der Winkel zwischen Unterschenkel und Vorfuß) sehr klein gegenüber dem plantaren Schwenkwinkel (das ist der Winkel zwischen Unterschenkel und Ferse). Außerdem sind der mediale (innere) Schwenkwinkel und der laterale (äußere) Schwenkwinkel unterschiedlich. Durch die erfindungsgemäße Ausbildung werden die positiven Eigenschaften eines Gelenkfußes beim Fersenauftritt mit den positiven Eigenschaften eines gelenklosen Fußes beim Abrollen über den Vorfuß miteinander vereinigt. Außerdem ist eine begrenzte Torquierung um die Beinlängsachse möglich.

Mit dem bei der Aufgabenstellung verwendeten Begriff "komfortbetont" wird erfindungsgemäß eine Charakteristik umschrieben, die sich vor allem durch angenehm weiches Abrollen in der gesamten Standphase des Prothesenschrittes vom Fersenauftritt bis zum Abheben der Fußspitze auszeichnet. Ein komfortbetontes Verhalten des Prothesenfußes beim Fersenauftritt bedeutet z. B. für einen Oberschenkelamputierten eine möglichst geringe Fersensteifigkeit, die einerseits bei Kompression des Fersenkeils noch ausreichende Stoßabsorbtion bietet, andererseits aber mit fortschreitendem Gang ein das Sicherheitsgefühl des Amputierten förderndes zügiges, jedoch nicht hartes Aufsetzen des Vorfußes ermöglicht, ohne daß hierbei zur Kompensation der kniebeugenden Wirkung der vom Fuß auf den Unterschenkel übertragenen Rückstellkraft ein nennenswertes Hüftstreckmoment generiert werden muß.

Bei einem künstlichen Fuß gemäß der gattungsbildenden DE-PS 36 44 613 führt ein Über- bzw. Unterschreiten der maximalen Größe bzw. der minimalen Steifigkeit des Fersenkeils zwar bei vergleichsweise geringer Fersenbelastung zum erwünschten sanften Aufsetzen des Vorfußes. Hierbei nimmt der Prothesenfuß jedoch eine gegenüber seiner Neutralstellung beim Stehen so stark nach hinten abfallende Position ein, daß der Amputierte das Gefühl hat, in ein Loch getreten zu sein, aus dem herauszukommen einen Energieaufwand erfordert, den er auf Dauer kaum aufzubringen vermag. Demgegenüber reagiert der erfindungsgemäße Fuß auf Fersenbelastung grundsätzlich mit einer Plantarflexion genannten Verkleinerung des Winkels zwischen Unterschenkel und Ferse bis zum Aufsetzen des Vorfußes, wobei der Prothesenfuß eine Position einnimmt, die seiner Neutralstellung beim Stehen genau entspricht. Das weitere Überrollen über den Vorfuß ist deshalb im Unterschied zu einem gelenklosen Prothesenfuß mit großem und weichem Fersenkeil mit geringerem Energieaufwand möglich.

Bei einer konventionellen Gelenkkonstruktion liegt die Gelenkachse im Fuß sehr hoch, häufig in der Ebene der Fußanschlußfläche, und weist von dem vertikal wirkenden Plantarpuffer nur einen kurzen Hebelarm auf. Da bei der erfindungsgemäßen Lösung der Drehpunkt des Gelenkes im Fuß sehr tief angeordnet ist (er liegt vorzugsweise im unteren Viertel der Fußhöhe), ergibt sich gegenüber dem im Bereich der Fußanschlußfläche angeordneten, horizontal wirkenden Plantarpuffer ein erheblich längerer Hebelarm, so daß bei gleichem Plantarmoment bei der erfindungsgemäßen Konstruktion die Kraft des Plantarpuffers kleiner sein kann. Der erfindungsgemäße Plantarpuffer kann daher weicher ausgebildet sein, was sich auch positiv auf seine Abmessungen auswirkt. Aufgrund des tief angeordneten Gelenkpunktes ergibt sich auch noch ein kosmetischer Vorteil: Der bei vielen konventionellen Lösungen typische Gelenkspalt hinter der Gelenkachse in Neutralstellung und vor der Gelenkachse bei Plantarflexion entfällt bei der erfindungsgemäßen Lösung vollständig.

Im Stand (Zustand des aufrechten Stehens) liegt das Lot aus dem Körperschwerpunkt des Prothesenträgers bei seitlicher (sagittaler) Betrachtung des Stehenden vor dem Gelenkdrehpunkt des Fußes und erzeugt somit über den elastischen Vorfuß ein das Knie sicherndes Moment, soweit auch die übrigen Prothesenkomponenten entsprechend den Regeln des Prothesenaufbaus zueinander ausgerichtet sind.

Der erfindungsgemäße Fuß vermeidet im Fersenbereich ein starkes Einsinken mit dem hiermit verbundenen Verlust an Körperschwerpunkt-Höhe, wie es dem vorbekannten gelenklosen Fuß ei-gen ist. Der erfindungsgemäße Fuß nimmt nach dem Aufsetzen des Vorfußes eine waagerechte Position ein, während der gelenklose Fuß in eine stark nach hinten abfallende Position gerät. Weitere Vorteile des erfindungsgemäßen Fußes liegen in der wartungs- und nahezu verschleißfreien Ausbildung des Gelenkes. Am Fuß sind keine weiteren Montage- oder, Justierarbeiten erforderlich. Deshalb ist es erfindungsgemäß möglich, daß der Außenfuß auch auf der Fußunterseite vollständig geschlossen ausgebildet ist.

Weitere Merkmale der Erfindung sind Gegenstand der Unteransprüche und werden in Verbindung mit weiteren Vorteilen der Erfindung anhand von Ausführungsbeispielen näher erläutert.

In der Zeichnung sind einige als Beispiele dienende Ausführungsformen der Erfindung dargestellt. Es zeigen:
- **Figur 1** -: einen Längsschnitt durch einen künstlichen, mit einem Gelenk versehenen Fuß;
- **Figur 2** -: einen Querschnitt gemäß der Linie II/II in **Figur 1**;
- **Figur 3** -: in vergrößertem Maßstab in einer Darstellung gemäß **Figur 1** eine abgewandelte Ausführungsform und
- **Figur 4** -: eine schematische Darstellung zur Veranschaulichung der Wirkung eines erfindungsgemäßen Plantarpuffers auf einen Bolzen des Fußgelenkes.

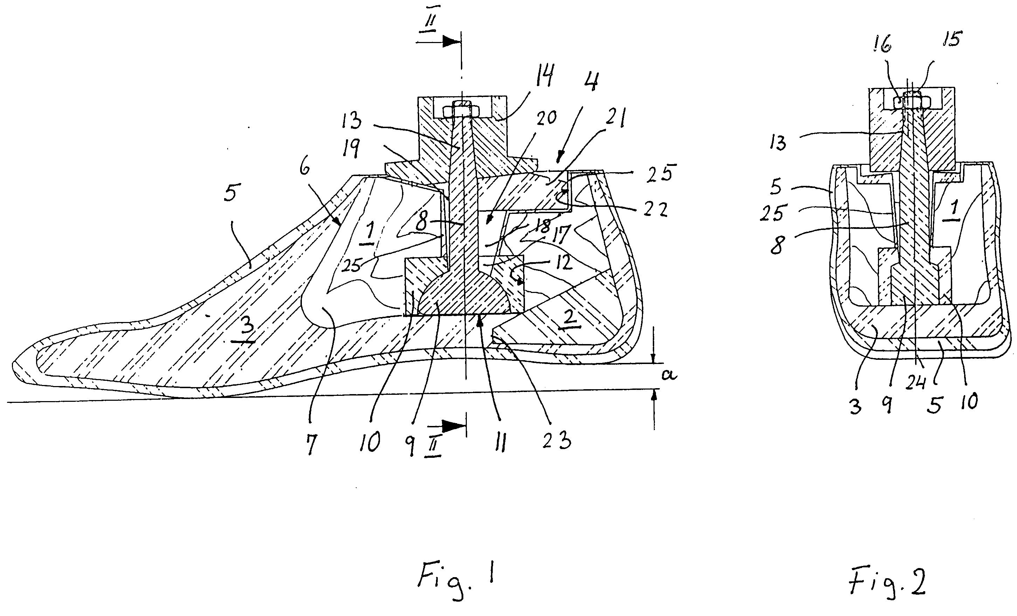

Der in den Figuren 1 bis 3 dargestellte künstliche Fuß besteht im wesentlichen aus einem Kern 1, einem sich an dessen Unterseite anschließenden Fersenkeil 2, einem Innenfuß 3 und einem diese Teile mit Ausnahme einer oberen Anschlußfläche 4 vollständig umgebenden Außenfuß 5. Vorgesehen ist ferner ein allseitig bewegliches, tiefliegendes Gelenk.

Der nahezu inkompressible Kern 1 besteht vorzugsweise aus Pappelholz mit einer spezifischen Masse von z. B. 0,48 g/cm³. Der Kern 1 erstreckt sich mit seiner an die Anschlußfläche 4 anschließenden Vorderseite 6 über einen nach vorn in der Höhe abnehmenden Ansatz 7 in den Spannbereich des Fußes. Die Gesamtlänge des Kernes 1 entspricht vorzugsweise 50 % bis 60 % der gesamten Fußlänge.

Das Gelenk weist einen angenähert in der Lotrechten stehenden Bolzen 8 auf, dessen unteres Ende als Gelenkkopf 9 ausgebildet ist, und oben sowie seitlich von einer Lagerschale 10 übergriffen wird. Der Gelenkkopf 9 ist durch eine halbkreisförmige Scheibe gebildet, die in der Längsmittelebene des Fußes liegt und mit ihrer Flachseite 11 auf einem ebenen Abschnitt des Innenfußes 3 aufliegt. Die Lagerschale 10 ist ein Kunststoffteil mit einer Härte von 80 bis 90 Shore und ist in eine entsprechende, nach unten hin offene Ausnehmung 12 im Kern 1 eingesetzt und schließt mit der Unterseite des Kerns 1 angenähert bündig ab. Das obere Ende des Bolzens 8 ist als Konus 13 ausgebildet, der in einen entsprechenden Innenkonus eines Adapters 14 gesteckt ist und an seinem freien Ende ein Spanngewinde 15 trägt, auf das eine sich auf dem Adapter 14 abstützende Spannmutter 16 aufgeschraubt ist. Der Bolzen 8 ist von unten durch eine Durchtrittsöffnung 17 in der Lagerschale 10 und durch eine sich hieran anschließende Durchtrittsöffnung 18 im Kern 1 gesteckt. Die beiden miteinander fluchtenden Durchtrittsöffnungen 17,18 erweitern sich nach oben konisch derart, daß bei lotrecht ausgerichtetem Bolzen 8 zwischen dessen vorderem Umfangsbereich und den vorderen Begrenzungen der Durchtrittsöffnungen 17,18 ein schmaler lichter Keilabstand 19 und zwischen dem hinteren Bolzen-Umfangsbereich und den hinteren Begrenzungen der Durchtrittsöffnungen 17,18 ein breiterer lichter Keilabstand 20 verbleibt.

Der Bolzen 8 stützt sich mit seiner der Ferse zugewandten Rückseite an einem horizontal gerichteten Plantarpuffer 21 ab, der in eine zur Anschlußfläche 4 und nach vorne offene Ausnehmung 22 im Kern 1 eingelegt ist und den Bolzen 8 nach Art einer im wesentlichen horizontal wirkenden Druckfeder beaufschlagt. Dabei wird der Plantarpuffer 21 durch ein Kunststoffteil gebildet, das eine spezifische Masse von etwa 0,4 bis 0,5 g/cm³ aufweist. Der Adapter 14 stützt sich mit seiner Unterseite in dem vor dem Bolzen 8 liegenden vorderen Bereich direkt oder indirekt auf dem Kern 1 und mit dem hinter dem Bolzen 8 liegenden hinteren Bereich auf dem Plantarpuffer 21 ab, der mit seiner Oberseite zum hinteren Ende des Fußes hin etwas ansteigt.

Der Fersenkeil 2 besteht aus weichem Kunststoffschaum mit einer spezifischen Masse von etwa 0,2 bis 0,4 g/cm³, steigt nach hinten an auf eine etwa im Bereich des Gelenkkopfes 9 liegende Höhe und erstreckt sich mit seiner Vorderseite 23 bis unter den hinteren Bereich der Lagerschale 10 und bis maximal zur Bolzenachse 24.

Der Innenfuß 3 besteht aus einem Kunststoffschaum mit einer spezifischen Masse von etwa 0,5 bis 0,8 g/cm³, schließt sich an die Vorderseite 6 des Kernes 1 und an die Vorderseite 23 des Fersenkeils 2 an, erstreckt sich bis in den Zehenbereich hinein und deckt mit einer dünnen Umhüllung 25 die obere Anschlußfläche 4, die Ausnehmung 22 für den Plantarpuffer 21 sowie die Wandungen der Durchtrittsöffnung 18 im Kern 1 ab. Bei der Herstellung des Fußes wird zuerst die Lagerschale 10 in die ihr zugeordnete Ausnehmung 12 im Kern 1 eingesetzt. Anschließend wird der Bolzen 8 von unten eingeschoben, bis der Gelenkkopf 9 gegen die Lagerschale 10 anliegt. Dann wird der Fersenkeil 2 angesetzt und die Teilekombination aus Kern 1, Lagerschale 10 mit Gelenkkopf 9 und Fersenkeil 2 zur Bildung des Innenfußes 3 umschäumt, wobei sich auch die vorstehend beschriebene dünne Umhüllung 25 bildet. Kern 1 und Fersenkeil 2 sind somit allseitig vom Innenfuß 3 abgedeckt mit Ausnahme der Anlagefläche zwischen Kern 1 und Fersenkeil 2.

Der Außenfuß 5 wird gebildet durch eine weiche, leicht verformbare, hautbildende Kunststoffschaumschicht, deren Rückstellkräfte klein sind gegenüber den Rückstellkräften des Innenfußes 3, und die eine spezifische Masse von etwa 0,4 bis 0,6 g/cm³ aufweist. Dieser Außenfuß 5 umschließt vollständig den Innenfuß 3 und läßt lediglich die Anschlußfläche 4 offen.

Figur 1 läßt erkennen, daß der Bolzen 8 nur sehr wenig nach vorn, aber erheblich weiter nach hinten verschwenken kann, wobei die Verschwenkung jeweils unter einer durch die Flachseite 11 des Gelenkkopfes 9 bewirkten elastischen Verformung des Innenfußes 3 und die Verschwenkung nach hinten zusätzlich gegen die elastische Federkraft des Plantarpuffers 21 erfolgt. Die Plantarflexion in der Sagittal-Ebene, also die Verschwenkung des Bolzens 8 innerhalb der Zeichenebene der Figur 1, beträgt maximal 15°. Die Sagittal-Ebene ist eine lotrecht in der Gehrichtung liegende Ebene. Durch die Elastizität der den Gelenkkopf 9 umgebenden Materialien der Lagerschale 10 und des Innenfußes 3 ist auch eine Gelenk-Querbewegung in einer lotrechten, quer zur Gehrichtung liegenden Frontalebene gegeben, wobei der Schwenkbereich in lateraler Richtung kleiner ist als der in medialer Richtung. Durch die Elastizität des die Lagerschale 10 bildenden Materials wird auch eine Torsion des Fußes um die Beinlängsachse ermöglicht.

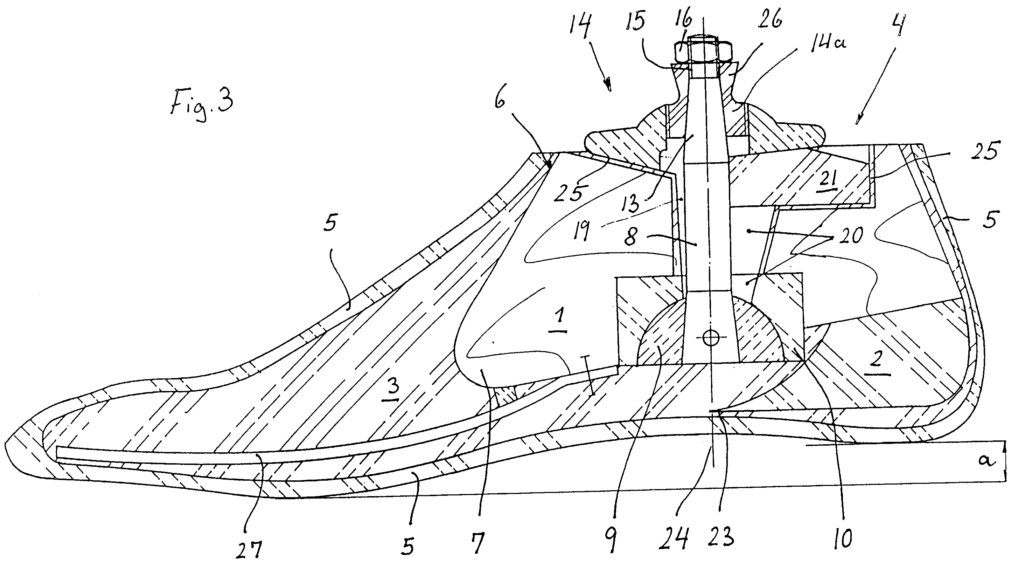

Figur 3 zeigt einen gegenüber Figur 1 in einigen Details abgewandelten künstlichen Fuß, wobei jede der Modifikationen für sich allein Verwendung finden kann.

Eine erste Abwandlung liegt in der zweiteiligen Ausbildung des Adapters 14, in den ein zentrisches Teil 14a eingesetzt ist, das mit dem Innenkonus für den Konus 13 des Bolzens 8 versehen ist und mit einem pyramidenförmigen Kupplungsteil 26 für eine justierbare Verbindung mit dem Unterschenkel bestückt ist. Eine entsprechende justierbare Verbindung zeigt
z. B. die DE-PS 19 22 619.

Eine weitere Modifikation liegt in der zweiteiligen Ausbildung von Bolzen 8 und Gelenkkopf 9, die durch einen Scherstift oder dergleichen drehfest miteinander verbunden sind.

Eine weitere Modifikation liegt in der zusätzlichen Anordnung eines Verstärkungsgurtes 27, der durch einen Kunststoffstreifen gebildet sein kann und an der Unterseite des vorderen Ansatzes 8 des Kerns 1 befestigt ist und sich bis in den Zehenbereich hinein erstreckt. Durch diese Vorfußverstärkung kann der Gefahr einer Rißbildung an der Fußsohle durch Längsüberdehnung entgegengewirkt werden, die sich bei Verwendung von Kunststoffschäumen mit spezifischen Gewichten an der untersten Grenze des beanspruchten Bereiches ergeben könnte.

Der künstliche Fuß gemäß den dargestellten Ausführungsbeispielen wird üblicherweise mit einem nicht näher dargestellten Schuh getragen, dessen Absatzhöhe a in den Figuren 1 und 3 eingetragen ist.

Die wesentlichen Vorteile des erfindungsgemäßen Fußes liegen in der Kombination der positiven Eigenschaften eines Gelenkfußes beim Fersenauftritt mit den positiven Eigenschaften eines gelenklosen Fußes beim Abrollen über den Vorfuß, in einer differenzierten Querbeweglichkeit in medialer und lateraler Richtung sowie in einer begrenzten Torquierung um die Beinlängsachse.

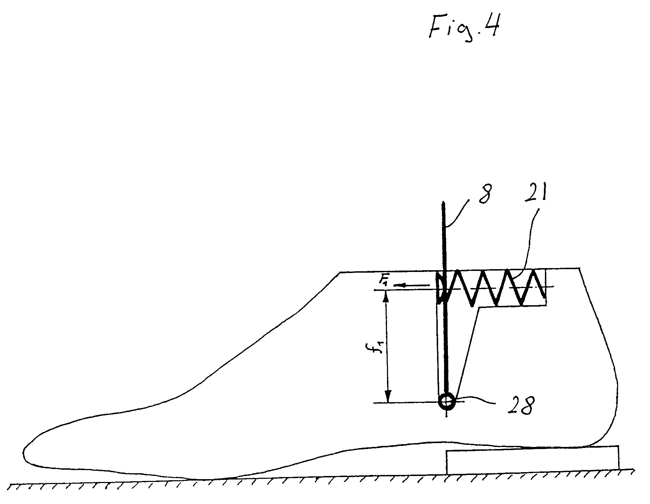

Figur 4 verdeutlicht das auf den Bolzen 8 ausgeübte Plantarmoment: Der Plantarpuffer 21 ist als horizontal liegende Druckfeder dargestellt, die sich mit ihrem hinten liegenden Ende an der die Ausnehmung 22 rückseitig begrenzenden Wandung und mit seinem vorderen Ende am Bolzen 8 abstützt. Die Federkraft des Plantarpuffers 21 ist mit F₁ angegeben, während der Hebelarm zu der schematisch angedeuteten Gelenkachse 28 mit f₁ bezeichnet ist. Gegenüber konventionellen Lösungen kann somit aufgrund des erheblich längeren Hebelarmes f₁ die Kraft F₁ des Plantarpuffers 21 zur Erzeugung des gleichen Plantarmomentes kleiner sein; der Plantarpuffer 21 kann also weicher ausgebildet werden.

Die Figur 4 zeigt die tatsächlichen Verhältnisse nur in vereinfachter Form. Denn bei einer Verschwenkung nach hinten erfolgt die Verschwenkung des am Plantarpuffer 21 anliegenden Abschnitts des Bolzens 8 auf einem Kreisbogen um den Gelenkmittelpunkt, wobei dann die Wirkung des Plantarpuffers im komprimierten Zustand nicht exakt horizontal, sondern tangential zu diesem Kreisbogen gerichtet ist. Diese Abweichung ist aber so gering, daß in der schematischen Darstellung von einer im wesentlichen horizontal wirkenden Druckfeder ausgegangen werden kann.

## Patentansprüche

1. Künstlicher Fuß mit
- einem praktisch inkompressiblen, im Knöchelbereich eine obere Anschlußfläche (4) bildenden Kern (1), der sich mit seiner an die Anschlußfläche (4) anschließenden Vorderseite (6) über einen nach vorn in der Höhe abnehmenden Ansatz (7) in den Spannbereich des Fußes erstreckt und insgesamt etwa halb so lang ist wie der gesamte Fuß;
- einem sich an die Unterseite (6) des Kernes (1) anschließenden Fersenkeil (2) aus weichem Kunststoffschaum;
- einem sich an die Vorderseite (6) des Kernes (1) und an die Vorderseite (23) des Fersenkeils (2) anschließenden Innenfuß (3), der sich bis in den Zehenbereich erstreckt und aus einem Kunststoffschaum besteht, und mit
- einem den Innenfuß (3) mit Ausnahme der oberen Anschlußfläche (4) vollständig umgebenden Außenfuß (5), der aus einer weichen, leicht verformbaren, hautbildenden Kunststoffschaumschicht besteht, deren Rückstellkräfte klein sind gegenüber den Rückstellkräften des Innenfußes (3),
**gekennzeichnet durch** folgende Merkmale:
a) ein allseitig bewegliches, tiefliegendes Gelenk (8,9,10,11);
b) das Gelenk weist einen angenähert in der Lotrechten stehenden Bolzen (8) auf, dessen unteres Ende als Gelenkkopf (9) ausgebildet ist, der auf seiner Oberseite sowie seitlich von einer Lagerschale (10) übergriffen wird;
c) der Bolzen (8) stützt sich mit seiner der Ferse zugewandten Rückseite an einem horizontal gerichteten Plantarpuffer (21) ab, der in eine zur Anschlußfläche (4) und nach vorne offene Ausnehmung (22) im Kern (1) eingelegt ist und den Bolzen (8) nach Art einer im wesentlichen horizontal wirkenden Druckfeder beaufschlagt;
d) das obere Ende des Bolzens (8) ist an einem Adapter (14) befestigt, der sich mit seiner Unterseite in dem vor dem Bolzen (8) liegenden vorderen Bereich direkt oder indirekt auf dem Kern (1) und mit dem hinter dem Bolzen (8) liegenden hinteren Bereich auf dem Plantarpuffer (21) abstützt.

2. Künstlicher Fuß nach Anspruch 1, **dadurch gekennzeichnet**, daß sich der Fersenkeil (2) mit seiner Vorderseite (23) nur bis unter den Gelenkbereich erstreckt.

3. Künstlicher Fuß nach Anspruch 2, **dadurch gekennzeichnet,** daß sich der Fersenkeil (2) mit seiner Vorderseite (23) bis maximal zur Bolzenachse (24) erstreckt.

4. Künstlicher Fuß nach Anspruch 1, 2 oder 3, **dadurch gekennzeichnet**, daß die Lagerschale (10) in eine entsprechende, nach unten hin offene Ausnehmung (12) im Kern (1) eingesetzt ist und mit der Unterseite des Kerns (1) angenähert bündig abschließt.

5. Künstlicher Fuß nach einem der vorhergehenden Ansprüche, **dadurch gekennzeichnet**, daß der Bolzen (8) von unten durch eine Durchtrittsöffnung (17) in der Lagerschale (10) und durch eine sich hieran anschließende Durchtrittsöffnung (18) im Kern (1) gesteckt ist, wobei die beiden miteinander fluchtenden Durchtrittsöffnungen (17,18) sich nach oben konisch erweitern derart, daß bei lotrecht ausgerichtetem Bolzen (8) zwischen dessen vorderen Umfangsbereich und den vorderen Begrenzungen der Durchtrittsöffnungen (17,18) ein schmaler lichter Keilabstand (14), zwischen dem hinteren Bolzen-Umfangsbereich und den hinteren Begrenzungen der Durchtrittsöffnungen (17,18) hingegen einer breiterer lichter Keilabstand (20) verbleibt.

6. Künstlicher Fuß nach einem der vorhergehenden Ansprüche, **dadurch gekennzeichnet**, daß der Gelenkkopf (9) durch eine halbkreisförmige Scheibe gebildet ist, die in der Längsmittelebene des Fußes liegt und mit ihrer Flachseite (11) auf einem ebenen Abschnitt des Innenfußes (3) aufliegt.

7. Künstlicher Fuß nach einem der vorhergehenden Ansprüche, **dadurch gekennzeichnet**, daß das obere Ende des Bolzens (8) als Konus (13) ausgebildet ist, der in einen entsprechenden Innenkonus des Adapters (14) gesteckt ist und an seinem freien Ende ein Spanngewinde (15) trägt, auf das eine sich auf dem Adapter (14) abstützende Spannmutter (16) aufgeschraubt ist.

8. Künstlicher Fuß nach einem der vorhergehenden Ansprüche, **dadurch gekennzeichnet**, daß der Adapter (14) ein pyramidenförmiges Kupplungsteil (26) für eine justierbare Verbindung mit dem Unterschenkel aufweist. **(Figur 3)**

9. Künstlicher Fuß nach einem der vorhergehenden Ansprüche, **dadurch gekennzeichnet**, daß der Plantarpuffer (21) ein Kunststoffteil mit einer spezifischen Masse von 0,4 bis 0,5 g/cm³ ist.

10. Künstlicher Fuß nach einem der vorhergehenden Ansprüche, **dadurch gekennzeichnet**, daß der Fersenkeil (2) eine spezifische Masse von 0,2 bis 0,4 g/cm³ aufweist.

11. Künstlicher Fuß nach einem der vorhergehenden Ansprüche, **dadurch gekennzeichnet**, daß der Innenfuß (3) eine spezifische Masse von 0,5 bis 0,8 g/cm³ aufweist.

12. Künstlicher Fuß nach einem der vorhergehenden Ansprüche, **dadurch gekennzeichnet**, daß der Außenfuß (5) eine spezifische Masse von 0,4 bis 0,6 g/cm³ aufweist.

13. Künstlicher Fuß nach einem der vorhergehenden Ansprüche, **dadurch gekennzeichnet**, daß die Lagerschale (10) ein Kunststoffteil mit einer Härte von 80 bis 90 Shore ist.

14. Künstlicher Fuß nach einem der vorhergehenden Ansprüche, **dadurch gekennzeichnet**, daß die Plantarflexion in der Sagittal-Ebene maximal 15° beträgt.

15. Künstlicher Fuß nach einem der vorhergehenden Ansprüche, **dadurch gekennzeichnet**, daß durch die Elastizität der den Gelenkkopf (9) umgebenden Materialien der Lagerschale (10) und des Innenfußes (3) eine Gelenk-Querbewegung in einer lotrechten, quer zur Gehrichtung liegenden Frontalebene gegeben ist.

16. Künstlicher Fuß nach Anspruch 15, **dadurch gekennzeichnet**, daß der Schwenkbereich nach lateral kleiner ist als der nach medial.

17. Künstlicher Fuß nach einem der vorhergehenden Ansprüche, **dadurch gekennzeichnet**, daß durch die Elastizität des die Lagerschale (10) bildenden Materials eine Torquierung des Fußes um die Beinlängsachse gegeben ist.

18. Künstlicher Fuß nach einem der vorhergehenden Ansprüche, **dadurch gekennzeichnet**, daß an der Unterseite des vorderen Ansatzes (8) des Kerns (1) ein sich bis in den Zehenbereich erstreckender Verstärkungsgurt (27) befestigt ist. **(Figur 3)**

19. Künstlicher Fuß nach einem der vorhergehenden Ansprüche, **dadurch gekennzeichnet**, daß der Innenfuß (3) vollständig um den Kern (1) und den Fersenkeil (2) geschäumt ist.

20. Künstlicher Fuß nach einem der vorhergehenden Ansprüche, **dadurch gekennzeichnet**, daß der Innenfuß (3) mit einer dünnen Umhüllung (25) die obere Anschlußfläche (4), die Ausnehmung (22) für den Plantarpuffer (21) sowie die Wandungen der Durchtrittsöffnung (18) im Kern (1) abdeckt.

21. Künstlicher Fuß nach einem der vorhergehenden Ansprüche, **dadurch gekennzeichnet**, daß der Außenfuß (5) auch auf der Fußunterseite vollständig geschlossen ausgebildet ist.
